# EUROPEAN PATENT APPLICATION

(11) **EP 4 032 529 A1**
(43) Date of publication of application: **27.07.2022**
(21) Application number: 20946527.7
(22) Date of filing: 07.12.2020
(51) Int. Cl.: A61K 9/20, A61K 31/44, A61K 47/34, A61K 47/38, A61P 35/00

(54) **SORAFENIB PHARMACEUTICAL COMPOSITION HAVING HIGH BIOAVAILABILITY AND APPLICATION**

(71) Applicant: Tianjin Creatron Biotechnology Co., Ltd., Tianjin 300457 (CN); Beijing Creatron Institute of Pharmaceutical Research Co., Ltd., Beijing 102200 (CN)
(72) Inventor: JIA, Huijuan, 102200 Beijing (CN); ZHANG, Jiayan, 102200 Beijing (CN); HOU, Xin, 102200 Beijing (CN); LI, Yan, 102200 Beijing (CN)
(74) Representative: IPrime Rentsch Kaelin AG
(86) International application number: PCT/CN2020/134164
(87) International publication number: WO 2022/120512

(57) **Abstract**

The present disclosure provides a sorafenib pharmaceutical composition with high bioavailability and use thereof, and specifically provides a low-dose sorafenib oral solid preparation, comprising: a) a sorafenib solid dispersion; b) a crystallization inhibitor; and c) additional pharmaceutically acceptable adjuvant. The aforementioned low-dose sorafenib oral solid preparation provided by the present disclosure has high bioavailability and reduces the dosage of sorafenib that the same therapeutic effect as that of Nexavar tablet can be achieved when a patient takes orally 35% to 70% of the administered dose of Nexavar tablet; it has higher stability, better safety, and less incidence of side effects; it has lower Cₘₐₓ and AUCo-t variation, a higher dissolution, and a low crystal precipitation rate with the increase of pH in the gastrointestinal tract; it is easy to be taken by patients due to the small volume of the tablet; it has a fast disintegration speed and a good dissolution effect; and it is easy to realize industrialization.

## Description

### FIELD

The present disclosure relates to the technical field of pharmaceutical preparations, in particular to a sorafenib pharmaceutical composition with high bioavailability and use thereof.

### BACKGROUND

Nexavar sorafenib tablet is the first multi-target oral tumor-targeted drug jointly developed by Bayer Company, Germany and Onyx Company for the treatment of advanced liver cancer, renal cell carcinoma and thyroid cancer. Sorafenib can act on tumor cells and blood vessels in the tumor at the same time, and play a role in inhibiting the growth of tumors through mechanisms such as anti-cell proliferation, promoting cell apoptosis, and inhibiting angiogenesis. Sorafenib, on the one hand, exerts an anti-cell proliferation effect by inhibiting c-Raf kinase and downstream signaling, blocking the phosphorylation process of MEK and ERK, and reducing the phosphorylation level of ERK. On the other hand, sorafenib inhibits the autophosphorylation process of tyrosine kinase receptor and thus exerts an anti-angiogenic effect by inhibiting vascular endothelial cell growth factor receptor-2 (VEGFR-2), vascular endothelial cell growth factor receptor-3 (VEGFR-3) and platelet-derived growth factor receptor-β (PDGFR-β). It has a dual anti-tumor effect: it can directly inhibit the proliferation of tumor cells by blocking the cell signaling pathway mediated by RAF/MEK/ERK, and it can also block the formation of tumor neovascularization by inhibiting VEGFR and platelet-derived growth factor (PDGF) receptors. In addition, it inhibits the phosphorylation process of eukaryotic initiation factor-4E (eIF4E), down-regulates the level of anti-apoptotic protein Mcl-1 *in vivo,* to play a role in promoting cell apoptosis.

Sorafenib is a bisaryl urea oral multikinase inhibitor, which has a chemical name of 4-{4-[3-(4-chloro-3-trifluoromethyl-phenyl)acylurea]-phenoxy}-pyridine-2-carboxylic acid methylamine with a relative molecular mass of 464.8 and a structural formula as shown in formula (1). The active ingredient used in Nexavar sorafenib tablet is the tosylate of sorafenib, which has a molecular formula of C₂₁H₁₆ClF₃N₄O₃·C₇H₈O₃S, as shown in formula (2).

The structure of Sorafenib is as follows:

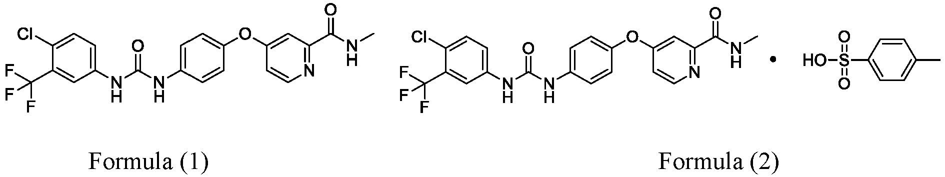

Sorafenib is a BCS class II drug with low solubility and high permeability according to the biopharmaceutics classification system. Sorafenib has a very low solubility in an aqueous solution of pH 1.2 to pH 7.4, which leads to a very low dissolution rate of sorafenib tablet in the gastrointestinal tract and becomes a rate-limiting step of absorption. Moreover, combined with the first-pass metabolism, sorafenib tablet has a relatively low bioavailability after oral administration, and its average relative bioavailability is only 38%-49%, compared with oral solution. Therefore, the specification of Nexavar sorafenib tablet is 200 mg, two tablets each time, twice a day, with a maximum daily dose of 800 mg. According to the instructions of Nexavar sorafenib tablet, after a healthy volunteer took orally 100 mg of ¹⁴C radiolabeled sorafenib, 77% of the administered dose was excreted through feces, in which the prototype drug accounted for above 51%, indicating that the oral bioavailability of Nexavar sorafenib tablet is low, and more than half of the administered dose of the drug is excreted to the outside of the body through feces due to solubility.

The clinical trial results of sorafenib disclosed by Oncologist 2007; 12:426-37 and Ann Oncol 2005; 16: 1688-94 show that the human pharmacokinetic parameters of sorafenib had high individual variability, and the coefficients of variation of Cmax and AUC were 33%-88% and 5%-83%, respectively, after oral administration of 200 mg or 400 mg twice a day. Oncotarget, 2017, Vol. 8, (26), pp: 43458-43469 discloses a clinical trial conducted on 94 patients with renal cell carcinoma, in which 74 people took orally 400 mg sorafenib tablet in two doses, another 18 people took orally 600 mg sorafenib tablet in three doses, and 2 people took orally 400 mg sorafenib tablet in one dose. After 2 weeks of treatment, the steady-state blood (plasma) concentration of sorafenib was 881 to 12,526 ng/mL, and the incidences of main adverse reactions were as follows: diarrhea (76.5%), hand-foot syndrome (68.99%), and fatigue (55.32%). These serious adverse reactions were all related to the blood concentration of sorafenib. Those patients with serious adverse reactions had blood concentrations of more than 10,000 ng/ml, and these serious adverse reactions were reduced with dose reduction or withdrawal, demonstrating that these serious side effects are all related to the administered dose. Invest New Drugs. 2012 April; 30(2): 524-528 evaluated the pharmacokinetic behavior of sorafenib in plasma and cerebrospinal fluid by intravenously injecting sorafenib into adult rhesus monkeys, in which a single intravenous injection of sorafenib into rhesus monkeys resulted in a maximum blood concentration of 0.00045 ~ 0.00058 µg/mL in cerebrospinal fluid and an AUC₀₋₂₄ₕ of 0.0048 to 0.0016 µg•h/mL, with little variation among individuals. In contrast, the blood concentration and AUC of adults and children after oral administration of sorafenib vary greatly between individuals, indicating that after oral administration, a variety of factors in the gastrointestinal tract affect the dissolution rate of the drug, and then affect the absorption of the drug, which is the main reason for the high variation of the drug.

Cancer Treatment Reviews, 2016 indicates that low dissolution rate is the main factor for low bioavailability, blood concentration, high variability in exposure dose, safety or ineffectiveness after oral administration of sorafenib tablet.

In the prior art, various methods have been tried and studied in order to improve the oral bioavailability of sorafenib tablet, but so far the core patents of Nexavar sorafenib tablet has basically been invalidated, and no new product has been commercialized.

In order to improve the drug dissolution of sorafenib, the prior art uses a polymer carrier to prepare a sorafenib solid dispersion pharmaceutical composition, which initially has a higher dissolution rate and dissolution, but due to the very low solubility of sorafenib (pH1, 0.0034 mg/ml; pH4.5, 0.00013 mg/ml), sorafenib crystallizes and precipitates rapidly with the extension of dissolution time, resulting in a sharp decrease in dissolution. In a dissolution experiment with a pH 6.8 phosphate buffer solution containing 0.1% SDS as the dissolution medium, the cumulative dissolution decreased to basically the same as or even lower than that of Nexavar sorafenib tablet at 37°C for 2h-4h, resulting in an insignificant increase in the bioavailability of the solid dispersion pharmaceutical composition *in vivo,* and it is impossible to greatly reduce the daily dosage of the drug.

In the prior art, there are also reports of adding a cosolvent, such as tocopherol compounds, polyol fatty acid esters, polyalkoxylated fatty alcohol ethers, and hydrogenated castor oil to a solid dispersion. Due to the low melting point of these cosolvents, on the one hand, it increases the difficulty of the preparation and production process, and on the other hand, it will lead to a decrease of the physical stability of the sorafenib solid dispersion. During the long-term storage of the pharmaceutical composition, the amorphous sorafenib with poor thermodynamic stability is easily induced by the cosolvent to accelerate crystallization and precipitation, thereby failing to achieve the purpose of greatly improving the bioavailability of the drug and reducing the administered dose.

Sorafenib itself has a poor stability and is easily degraded to produce CTF-aniline, a mutagenic impurity, and is more likely to produce CTF-aniline after being prepared into a solid dispersion. The structure of CTF-aniline is shown in formula (3):

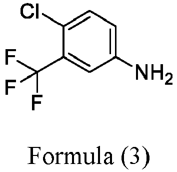

In addition, Nexavar sorafenib tablet has a large specification, and a large amount of polymer materials need to be added for the preparation into a solid dispersion, which makes it difficult to form the formulation. For example, the disintegration time of tablets and capsules is greatly prolonged, and in order to improve the disintegration, a large amount of preparation excipients need to be added, resulting in a large volume and weight of the contents in tablets or capsules, and the prepared preparation is not conducive to the patient's administration, resulting in poor compliance.

Due to the existence of the above problems, improved preparation products for Nexavar sorafenib tablet have been unable to enter the industrialization for clinical use.

### SUMMARY

In view of the above, the technical problem to be solved by the present disclosure is to provide a sorafenib pharmaceutical composition with high bioavailability and use thereof, and the prepared sorafenib pharmaceutical composition has a high dissolution and stability.

To achieve the above purpose, the present disclosure provides a sorafenib pharmaceutical composition with high bioavailability, comprising:
a) sorafenib, or a salt, hydrate, solvate, or a hydrate or solvate of the salt thereof; and
b) a carrier comprising VA64 and HPMCAS.

In some specific embodiments of the present disclosure, the sorafenib is a tosylate thereof, that is, sorafenib tosylate.

In the present disclosure, preferably, the mass ratio of the component a) to VA64 and HPMCAS in component b) is 1:(0.1~5):(0.01~5), more preferably 1:(0.5~3):(0.1~1).

More preferably, the mass ratio of the component a) to VA64 and HPMCAS in component b) is 1:1:0.1~1:1:0.5, further preferably 1:1:0.25.

In the present disclosure, preferably, the sorafenib pharmaceutical composition is a solid dispersion.

In the present disclosure, preferably, the solid dispersion is prepared according to a spray drying method, and the solvent of the spray drying method is a mixed solvent of methanol and dichloromethane.

In the present disclosure, preferably, the volume ratio of methanol and dichloromethane is 1:(1~4).

More preferably, it is 1:3.

In the present disclosure, a mixed solvent of methanol and dichloromethane is used as the solvent of the spray drying method to prepare the sorafenib solid dispersion, so that the prepared solid dispersion has better stability. Experiments show that using a mixed solvent of methanol and dichloromethane, especially a mixed solvent of methanol-dichloromethane with a volume ratio of 1:3, sorafenib has a higher solubility, and there is no significant change in related substances when placed in this organic solvent for 30 min and 24 h.

The weight volume ratio (w/v) of the component a) and the mixed solvent of methanol-dichloromethane is preferably 1:25~300, more preferably 1:50~150, and most preferably 1:100.

In the present disclosure, the inlet air temperature of the spray drying method is preferably 120°C, the outlet air temperature is preferably 60°C, and nitrogen is preferably used as a carrier gas.

The present disclosure also provides a low-dose sorafenib oral solid preparation, comprising: a) a sorafenib solid dispersion; b) a crystallization inhibitor; and c) additional pharmaceutically acceptable adjuvant.

In the present disclosure, preferably, the crystallization inhibitor is one or more selected from polyvinylpyrrolidone, hydroxypropyl methylcellulose acetate succinate (HPMCAS), sodium glycocholate, sodium taurocholate, sodium glycodeoxycholate, sodium glycochenodeoxycholate, sodium glycoursodeoxycholate, sodium taurodeoxycholate and sodium tauroursodeoxycholate, as well as sodium dodecyl sulfonate.

The present disclosure uses the above crystallization inhibitor, especially polyvinylpyrrolidone, to form a composition with the sorafenib solid dispersion to prepare a solid preparation, which ensures that the preparation has a good dissolution and avoids the crystallization and precipitation of sorafenib.

In the present disclosure, the polyvinylpyrrolidone is preferably PVPK30, PVPK25, PVPK60, PVPK90, PVPK12, PVPK15, PVPK17, more preferably PVPK30 and PVPK25.

In the present disclosure, preferably, the mass content of the polyvinylpyrrolidone is 1%~40%, more preferably 1%~20%, further preferably 10%~20%, and most preferably 12.5%.

In the present disclosure, the crystallization inhibitor may also be selected from hydroxypropyl methylcellulose acetate succinate (HPMCAS), including, but not limited to, one or more of HPMCAS HG (or the same specification, such as 126G), HPMCAS MG (or the same specification, such as 912 G), and HPMCAS LG (or the same specification, such as 716 G).

In the present disclosure, preferably, the mass content of the hydroxypropyl methylcellulose acetate succinate (HPMCAS) is 1%~40%, more preferably 1%~20%, and most preferably 1~10%.

In the present disclosure, the crystallization inhibitor may also be selected from a cholate compound, including, but not limited to: one or more of sodium glycocholate, sodium taurocholate, sodium glycodeoxycholate, sodium glycochenodeoxycholate, sodium glycoursodeoxycholate, sodium taurodeoxycholate and sodium tauroursodeoxycholate, as well as sodium dodecyl sulfonate, preferably any one or more of STC, SDC, and SGC.

In the present disclosure, preferably, the mass content of the cholate compound is 1%~40%, more preferably 1%~20%, and most preferably 1~10%.

In the present disclosure, preferably, the sorafenib solid dispersion comprises:
a) sorafenib, or a salt, hydrate, solvate, or a hydrate or solvate of the salt thereof; and
b) a carrier comprising VA64.

The present disclosure uses VA64 as a carrier of the sorafenib solid dispersion, and the prepared solid dispersion has higher stability.

In the present disclosure, preferably, the mass ratio of the component a) to VA64 is 1:(0.1∼5), more preferably 1:(0.5∼3), and further preferably 1:(1-3).

More preferably, the mass ratio of the component a) to VA64 is 1:1.

In the present disclosure, preferably, the sorafenib solid dispersion comprises:
a) sorafenib, or a salt, hydrate, solvate, or a hydrate or solvate of the salt thereof; and
b) a carrier comprising VA64 and HPMCAS.

The present disclosure uses VA64 and HPMCAS as a carrier of the sorafenib solid dispersion, and the prepared solid dispersion has higher stability and dissolution.

In the present disclosure, preferably, the mass ratio of the component a) to VA64 and HPMCAS is 1:(0.1~5):(0.01~5), more preferably 1:(0.5~3):( 0.1~1).

More preferably, the mass ratio of the component a) to VA64 and HPMCAS is 1:1:(0.1∼0.5), and further preferably 1:1:0.25.

In the present disclosure, preferably, the solid dispersion is prepared according to a spray drying method, and the solvent of the spray drying method is a mixed solvent of methanol and dichloromethane.

In the present disclosure, preferably, the volume ratio of methanol and dichloromethane is 1:(1~4).

More preferably, it is 1:3.

In the present disclosure, a mixed solvent of methanol and dichloromethane is used as the solvent of the spray drying method to prepare the sorafenib solid dispersion, so that the prepared solid dispersion has better stability. Experiments show that using a mixed solvent of methanol and dichloromethane, especially a mixed solvent of methanol-dichloromethane with a volume ratio of 1:3, sorafenib has a higher solubility, and there is no significant change in related substances when placed in this organic solvent for 30 min and 24 h.

The weight volume ratio (w/v) of the component a) and the mixed solvent of methanol-dichloromethane is preferably 1:25~300, more preferably 1:50~150, and most preferably 1:100.

In the present disclosure, the inlet air temperature of the spray drying method is preferably 120°C, the outlet air temperature is preferably 60°C, and nitrogen is preferably used as a carrier gas.

In the present disclosure, preferably, in the sorafenib oral solid preparation, the component a) has a unit dose of 70~200 mg, preferably 70~140 mg, and more preferably 70~130 mg.

In the present disclosure, the unit dose refers to the amount of the main drug contained in the smallest unit of the drug, such as the weight of the drug contained in one tablet, one capsule, or one bag of granules. For example, when the compound preparation is a tablet, the unit of the unit dose is mg/tablet.

In the present disclosure, the dosage of sorafenib is reduced by improving the stability and dissolution of sorafenib, and the same therapeutic effect as that of Nexavar tablet can be achieved when a patient takes orally 35%~70% of the administered dose of Nexavar tablet.

In the present disclosure, preferably, the adjuvant is one or more selected from a filler, disintegrant, binder, glidant, lubricant, and flavoring agent.

In the present disclosure, preferably, the filler is one or more selected from mannitol, pregelatinized starch, lactose, calcium hydrogen phosphate, starch, microcrystalline cellulose, pregelatinized starch, partially pregelatinized starch, magnesium sulfate, and calcium sulfate.

The mass content of the filler is preferably 20%-50%, more preferably 30%-40%.

In the present disclosure, preferably, the disintegrant is one or more selected from corn starch, partially pregelatinized starch, hydroxypropyl starch, carboxymethyl cellulose, sodium carboxymethyl cellulose, calcium carboxymethyl cellulose, sodium carboxymethyl starch, low-substituted hydroxypropyl cellulose, croscarmellose sodium, and crospovidone, more preferably crospovidone, sodium carboxymethyl starch or low-substituted hydroxypropyl cellulose.

The mass content of the disintegrant is preferably 3%-20%, more preferably 5%-10%.

In the present disclosure, preferably, the binder is one or more selected from hydroxypropyl cellulose, hydroxypropyl methylcellulose, povidone, starch slurry, and sodium carboxymethyl cellulose.

The mass content of the binder is preferably 0-10%, more preferably 0-5%.

In the present disclosure, preferably, the glidant is one or more selected from talc and silica; preferably silica.

The mass content of the silica is preferably 2%-20%, more preferably 5%-15%, and most preferably 10%.

In the present disclosure, by increasing the amount of silica, the disintegration of the preparation is accelerated, thereby improving the dissolution of the preparation.

In the present disclosure, preferably, the lubricant is one or more selected from magnesium stearate, stearic acid, calcium stearate, sodium stearyl fumarate, polyethylene glycol, hydrogenated vegetable oil, polyethylene glycol, sodium dodecyl sulfate, talc, and silica.

The mass content of the lubricant is preferably 0.3%-5%, more preferably 0.5%-2%, and most preferably 0.5%-1%.

In the present disclosure, preferably, the dosage form of the oral solid preparation is tablet, granule, dry suspension, capsule or film.

In the present disclosure, preferably, the above-mentioned oral solid preparation is prepared by a dry granulation process.

The present disclosure provides use of the above-mentioned sorafenib solid dispersion or the above-mentioned sorafenib oral solid preparation in the manufacture of a medicament for preventing, treating or alleviating liver cancer, renal cell carcinoma, and thyroid cancer.

The present disclosure provides a method for preventing, treating or alleviating liver cancer, renal cell carcinoma, and thyroid cancer, which comprises contacting the above-mentioned sorafenib solid dispersion or the above-mentioned sorafenib oral solid preparation with a biological sample.

Compared with the prior art, the present disclosure provides a low-dose sorafenib oral solid preparation, comprising: a) a sorafenib solid dispersion; b) a crystallization inhibitor; and c) additional pharmaceutically acceptable adjuvant. The aforementioned low-dose sorafenib oral solid preparation provided by the present disclosure has the following beneficial effects:
1. it has a higher bioavailability and reduces the dosage of sorafenib, and the same therapeutic effect as that of Nexavar tablet can be achieved when a patient takes orally 35%~70% of the administered dose of Nexavar tablet;
2. it has higher stability, better safety, and less incidence of side effects;
3. it has lower Cₘₐₓ and AUCo-t variation;
4. it has higher dissolution, which is 2-7 times higher than that of the marketed product "Nexavar" (calculated based on the area under the dissolution profile) when a pH 6.8 phosphate buffer solution containing 0.1% SDS (sodium dodecyl sulfate) is used as the dissolution medium;
5. it has a low crystal precipitation rate with the increase of pH in the gastrointestinal tract;
6. it is easy to be taken by patients due to the small volume of the tablet;
7. it has a fast disintegration speed and a good dissolution effect; and
8. it is easy to realize industrialization.

### BRIEF DESCRIPTION OF DRAWINGS

FIG. 1 shows the dissolution profile of tablets prepared with different ratios of VA64 solid dispersion in a pH 6.8 phosphate buffer solution (0.1% SDS); and
FIG. 2 shows the dissolution profile of tablets prepared with a composite carrier solid dispersion in a pH 6.8 phosphate buffer solution (0.1% SDS).

### DETAILED DESCRIPTION

In order to further illustrate the present disclosure, the sorafenib pharmaceutical composition with high bioavailability provided by the present disclosure and use thereof will be described in detail below in combination with examples.

### Example 1 Solubility of sorafenib in different organic solvents

An appropriate amount of sorafenib raw material was added to 25 ml of organic solvent and shaken in a shaker at room temperature for different times. Samples were taken to detect the amount of dissolved drug, and the results are shown in Table 1 below:

**Table 1 Solubility of sorafenib in different organic solvents**

| Organic solvent | Ratio (v/v) | Solubility (mg/mL)-37°C | |
|---|---|---|---|
| | | 30 min | 24 h |
| Methanol-dichloromethane | 1:0.5 | 8.34 | 19.17 |
| | 1:1 | 14.28 | 24.22 |
| | 1:2 | 20.90 | 36.17 |
| | 1:3 | 36.36 | 36.44 |
| | 1:4 | 31.31 | 36.32 |
| Acetone-ethanol | 1:2 | 18.51 | 18.48 |
| | 1:3 | Less API dissolved by visual inspection than 1:2 ratio | N/A |
| | 1:5 | | |
| Dichloromethane-acetone | 1:1 | Less API dissolved by visual inspection than 3:1 ratio | N/A |
| | 2:1 | | |
| | 3:1 | 3.60 | 4.04 |
| Isopropanol | N/A | 2.91 | 2.81 |

Related substances were detected by HPLC method, and the results are shown in Table 2 below:

**Table 2 Stability of sorafenib in different organic solvents**

| Organic solvent | Ratio | Standing time | Related substances (%) | | | | | | | | | |
|---|---|---|---|---|---|---|---|---|---|---|---|---|
| | | | RRT 0.03 | RRT0. 05 | RRT 0.07 | RRT 0.08 | RRT 0.30 | RRT 0.35 | RRT 0.40 | RRT 0.55 | RRT 0.92 | RRT 1.00 |
| API | | | \ | \ | 0.011 | 0.008 | 0.029 | \ | 0.006 | 0.028 | 0.054 | 99.864 |
| Methanol - dichloro methane | 1:2 | 30 min | \ | \ | 0.013 | 0.011 | 0.030 | \ | 0.008 | 0.031 | 0.037 | 99.870 |
| | | 24 h | \ | \ | 0.012 | 0.013 | 0.035 | \ | 0.009 | 0.028 | 0.049 | 99.853 |
| | 1:3 | 30 min | \ | \ | 0.013 | 0.015 | 0.029 | \ | 0.006 | 0.026 | 0.050 | 99.861 |
| | | 24 h | \ | \ | 0.011 | 0.012 | 0.035 | \ | 0.009 | 0.025 | 0.037 | 99.872 |
| | 1:4 | 30 min | \ | \ | 0.011 | 0.013 | 0.028 | \ | 0.007 | 0.024 | 0.034 | 99.884 |
| | | 24 h | \ | \ | 0.012 | 0.013 | 0.028 | \ | 0.008 | 0.032 | 0.045 | 99.862 |
| Acetone-ethanol | 1:2 | 30 min | \ | 4.314 | 0.005 | 0.011 | 0.029 | \ | 0.008 | 0.025 | 0.047 | 95.562 |
| | | 24 h | \ | 3.946 | 0.055 | 0.010 | 0.033 | \ | 0.007 | 0.020 | 0.035 | 95.893 |
| Dichloro methane-acetone | 3:1 | 30 min | \ | 12.97 9 | \ | 2.491 | 0.020 | 0.282 | \ | \ | 0.039 | 84.196 |
| | | 24 h | \ | 14.19 1 | 0.652 | \ | \ | \ | \ | \ | \ | 85.157 |
| Isopropa nol | N/A | 30 min | 0.161 | \ | \ | \ | \ | \ | \ | \ | \ | 99.839 |
| | | 24 h | \ | \ | 0.635 | \ | \ | \ | \ | \ | \ | 99.365 |

From the above results, it can be seen that sorafenib has a relatively large solubility in methanol-dichloromethane (1:3), and there is no significant change in related substances in terms of API when placed in this organic solvent for 30 min and 24 h. Therefore, methanol-dichloromethane is preferably used as the organic solvent for preparing a solid dispersion of sorafenib by a spray drying method.

### Example 2 Solubility and stability of a solid dispersion (SD)

### 1. Preparation of SD

Sorafenib (SLFN) /Sorafenib tosylate (TSSLFN) was added to a methanol-dichloromethane (1:3) mixed solvent, stirred and dissolved at 30-33°C. The main drug: the mixed solvent was 1:100. A carrier was then added and dissolved, and the carrier was used in an amount of the main drug: the carrier of 1:3.

Spray drying: the inlet air temperature was 120°C, the outlet air temperature was set to 60°C, and nitrogen was used as a carrier gas. The results are shown in Table 3.

**Table 3 DSC test results of solid dispersions prepared with different carriers and different ratios of carrier to main drug**

| Main drug | Carrier | Ratio | Endothermic peak of crystalline drugs in DSC curve |
|---|---|---|---|
| SLFN | No | -- | Endothermic peak at 208~220°C |
| SLFN | PVP K30 | 1:3 | No |
| SLFN | PVP K30 | 1:1 | No |
| SLFN | HPC LF | 1:3 | No |
| SLFN | HPMC E5 | 1:3 | No |
| SLFN | HPMC K100 | 1:3 | No |
| SLFN | HPMC AS 126G | 1:3 | No |
| SLFN | HPMC AS 716G | 1:3 | No |
| SLFN | VA64 | 1:3 | No |
| SLFN | VA64 | 1:1 | No |
| TSSLFN | No | -- | Endothermic peak at 180-200°C and/or 215~235°C |
| TSSLFN | HPMC E5 | 1:3 | No |
| TSSLFN | HPMC AS 716G | 1:3 | No |
| TSSLFN | VA64 | 1:3 | No |

According to the above results, the samples prepared with different carriers all formed solid dispersions.

### 2. Solubility test

An appropriate amount of solid dispersion (SD) was added to a pH 1.0 hydrochloric acid solution and a pH 6.8 phosphate buffer solution, while keeping the SD in excess, and shaken at 37°C in a shaker at 300 rpm. Samples were taken at different times to detect the content of sorafenib. The solid dispersions with VA64 and PVPK30 as carriers were investigated for the solubility in simulated intestinal fluid and simulated gastric fluid, and the results are as follows:

**Table 4 Solubility test results of sorafenib solid dispersion**

| Main drug | Carrier | Dissolution medium | Solubility (µg/mL) | | | | |
|---|---|---|---|---|---|---|---|
| | | | 0.5h | 1h | 2h | 3h | 4h |
| SLFN | / | pH1.0 hydrochloric acid solution (Main drug: Carrier 1:3) | <0.09 | \ | <0.09 | \ | <0.09 |
| TSSLFN | / | | 3.7 | \ | 4.1 | \ | 4.1 |
| SLFN | PVP K30 | | 11.2 | \ | 9.1 | \ | 3.3 |
| SLFN | HPC LF | | 7.8 | \ | 3.9 | \ | 3.1 |
| SLFN | HPMC E5 | | 16.7 | \ | 7.4 | \ | 3.4 |
| SLFN | HPMC K100 | | 12.5 | \ | 7.8 | \ | 3.6 |
| SLFN | HPMC AS 126G | | 7.1 | \ | 6.5 | \ | 4.2 |
| SLFN | HPMC AS 716G | | 7.5 | \ | 6.3 | \ | 3.9 |
| SLFN | VA64 | | 6.7 | \ | 5.9 | \ | 4.8 |
| TSSLFN | HPMC E5 | | 15.8 | \ | 8.3 | \ | 5.2 |
| TSSLFN | HPMC AS 716G | | 8.2 | \ | 8.4 | \ | 5.6 |
| TSSLFN | VA64 | | 6.9 | \ | 6.1 | \ | 5.3 |
| SLFN | / | pH6.8 phosphate buffer solution (Main drug: Carrier 1:3) | <0.09 | \ | <0.09 | \ | <0.09 |
| TSSLFN | / | | <0.09 | \ | <0.09 | \ | <0.09 |
| SLFN | PVP K30 | | <0.09 | \ | <0.09 | \ | <0.09 |
| SLFN | HPC LF | | <0.09 | \ | <0.09 | \ | <0.09 |
| SLFN | HPMC E5 | | <0.09 | \ | <0.09 | \ | <0.09 |
| SLFN | HPMC K100 | | <0.09 | \ | <0.09 | \ | <0.09 |
| SLFN | HPMC AS 126G | | <0.09 | \ | <0.09 | \ | <0.09 |
| SLFN | HPMC AS 716G | | 0.42 | \ | 0.39 | \ | 0.15 |
| SLFN | VA64 | | <0.09 | \ | <0.09 | \ | <0.09 |
| TSSLFN | HPMC E5 | | <0.09 | \ | <0.09 | \ | <0.09 |
| TSSLFN | HPMC AS 716G | | 0.41 | \ | 0.38 | \ | 0.11 |
| TSSLFN | VA64 | | <0.09 | \ | <0.09 | \ | <0.09 |
| SLFN | VA64 (1:1) | FaSSGF | 6.69 | \ | 7.24 | \ | \ |
| | | FaSSIF | \ | 349.85 | \ | 12.38 | \ |
| SLFN | PVPK30 (1:1) | FaSSGF | 7.05 | \ | 7.90 | \ | \ |
| | | FaSSIF | \ | 131.36 | \ | 3.60 | \ |

It can be seen from the above results that although the initial solubility (0.5h, 2h, 4h) of the solid dispersion in pH 1.0 hydrochloric acid medium increased, the solubility gradually decreased with the extension of time, and the crystalline drug of sorafenib appeared on the test tube wall, which was not much different from sorafenib tosylate at 24h. In a pH 6.8 phosphate medium, except for the solid dispersion with HPMC AS 716G as a carrier, the solubility of solid dispersions prepared by using other polymer materials as a carrier was lower than the limit of quantification.

There was no significant difference in the measured solubility of solid dispersions prepared with polymer VA64 and PVPK30 as a carrier in simulated gastric fluid (FaSSGF) for 30 min and 2 hours, which was about 7 µg/mL; the solubility of the VA64 carrier solid dispersion measured 1 hour after transferring to the simulated intestinal fluid (FaSSIF) was 350 µg/mL and rapidly decreased to 12.38 µg/mL after 2 hours; while the solubility of the solid dispersion with PVP K30 as a carrier measured 1 hour after transferring to the simulated intestinal fluid was 131 µg/mL and rapidly decreased to 3.6 µg/mL after 2 hours.

### 3. Determination of stability

The SD prepared above was sealed in an aluminum foil bag, placed in a high temperature of 60°C for investigation for 10 days and 30 days, and related substances were detected by HPLC method. The results are shown in Table 5 below.

**Table 5 Test results of influencing factors of sorafenib solid dispersion prepared with different carriers at a high temperature of 60°C**

| Investigation time | Main drug/carrier | Related substances (%) | | |
|---|---|---|---|---|
| | | Maximum single impurity | CTF -aniline | Total impurity |
| 0 day | SLFN | 0.03 | Not detected | 0.09 |
| 10 days | | 0.04 | Not detected | 0.10 |
| 30 days | | 0.05 | Not detected | 0.10 |
| 0 day | SLFN /PVP K30 | 0.09 | Not detected | 0.20 |
| 10 days | | 0.11 | Not detected | 0.23 |
| 30 days | | 0.12 | 0.01 | 0.32 |
| 0 day | SLFN /HPMC E5 | 0.04 | Not detected | 0.13 |
| 10 days | | 0.04 | Not detected | 0.13 |
| 30 days | | 0.06 | 0.02 | 0.23 |
| 0 day | SLFN /HPMC AS 716G | 0.04 | Not detected | 0.10 |
| 10 days | | 0.12 | 0.05 | 0.36 |
| 30 days | | 0.27 | 0.10 | 0.75 |
| 0 day | SLFN /VA64 | 0.04 | Not detected | 0.11 |
| 10 days | | 0.04 | Not detected | 0.12 |
| 30 days | | 0.03 | Not detected | 0.13 |
| 0 day | TSSLFN /VA64 | 0.03 | Not detected | 0.10 |
| 10 days | | 0.04 | Not detected | 0.12 |
| 30 days | | 0.04 | Not detected | 0.16 |
| 0 day | TSSLFN / HPMC AS 716G | 0.04 | Not detected | 0.11 |
| 10 days | | 0.12 | 0.06 | 0.41 |
| 30 days | | 0.29 | 0.11 | 0.83 |

From the above results, it can be seen that the SD prepared with VA64 as a carrier had the best chemical stability, while the solid dispersion with HPMCAS as a carrier had poor chemical stability; when a solid dispersion was prepared by using sorafenib free base and sorafenib tosylate as the main drug respectively and using the same ratio of the polymer carrier, the solid dispersion of sorafenib tosylate had slightly poor chemical stability after being placed at a high temperature of 60°C for 30 days.

### Example 3 Screening of carrier ratio

### 1. Screening of VA64 carrier ratio

Sorafenib solid dispersions with different carrier ratios (mass ratios) were prepared according to the above spray drying method, and DSC test was performed to confirm whether the solid dispersion was completely formed. The results are shown in Table 6.

**Table 6 DSC test results of solid dispersions prepared with different ratios of VA64 to the main drug**

| Main drug | Carrier | Ratio | Endothermic peak of crystalline drugs in DSC curve |
|---|---|---|---|
| SLFN | VA64 | 1:0.5 | Disappeared |
| SLFN | VA64 | 1:1 | Disappeared |
| SLFN | VA64 | 1:1.5 | Disappeared |
| SLFN | VA64 | 1:2 | Disappeared |

The sorafenib solid dispersions with above different carrier ratios were prepared into tablets according to the formula shown in Table 7:

**Table 7 Sorafenib solid dispersible tablet formula**

| Composition of the formula | Amount (mg/tablet) |
|---|---|
| SD amount (based on SLFN) | 100 |
| Silicified microcrystalline cellulose (SMCC) | 218.50 |
| Sodium dodecyl sulfate (SDS) | 4.50 |
| Croscarmellose sodium (CCNa) | 22.50 |
| Magnesium stearate (MS) | 4.50 |

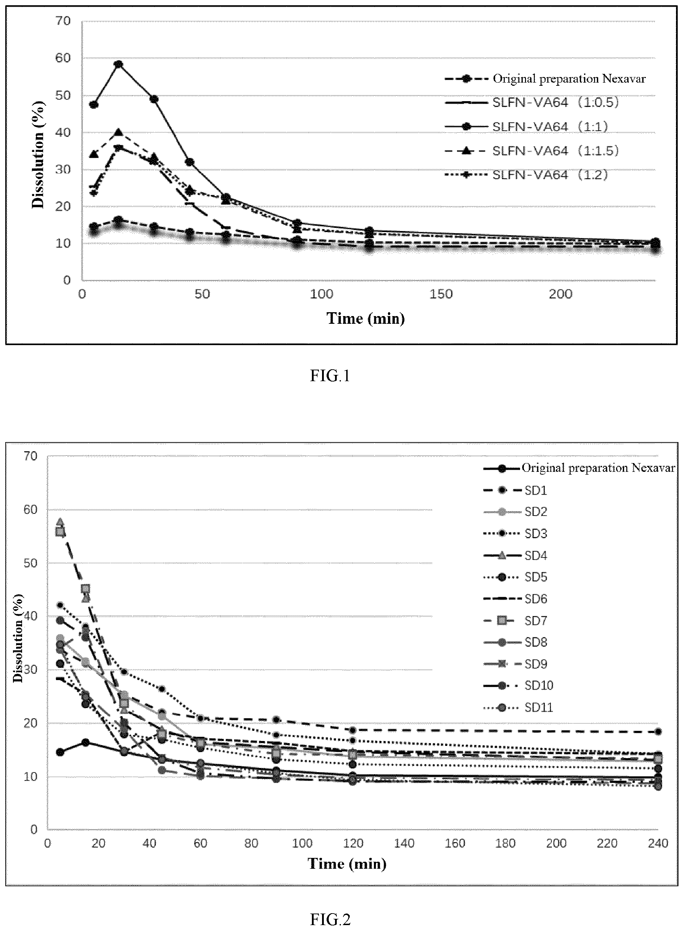

Raw and adjuvant materials in the amount shown in the formula were weighed. All raw and adjuvant materials except for MS were passed through a 50-mesh sieve 6 times, mixed, and granulated by a dry method. The weight of the granules was weighed, and the amount of MS added externally was calculated and weighed. They were mixed for 5 min and subjected to tableting. The dissolution profile was determined and shown in FIG. 1.

Dissolution method: paddle method, pH 6.8 phosphate buffer solution+ 0.1% SDS, 900 mL, 100 rpm, 37°C.

Sampling and sample treatment method: 10 mL of sample was taken and filtered with a PES filter membrane (diameter 25 mm, pore size 0.45 µm). 7 mL of filtrate was discarded, 2~3 mL of subsequent filtrate was taken, and 10 mL of medium was added to the dissolution cup. 1 ml of the filtered sample was transferred with a pipette into a 10 ml volumetric flask, and diluted by 10 times by adding a mobile phase to the scale mark.

**Table 8 Dissolution profile of sorafenib solid dispersion tablet (n=3)**

| Batch No. | Disint egratio n time min | Cumulative dissolution (%) | | | | | | | | AUC₀₋₄ₕ |
|---|---|---|---|---|---|---|---|---|---|---|
| | | 5 min | 15 min | 30 min | 45 min | 1h | 1.5h | 2h | 4h | |
| Nexavar (half tablet, 100 mg) | 4 | 14.6 | 16.4 | 14.6 | 13.1 | 12.4 | 11.1 | 10.2 | 9.9 | 2687.5 |
| SLFN-VA64 (1:0.5) | 38 | 25.3 | 36.2 | 31.7 | 20.8 | 14.2 | 10.3 | 9.2 | 9.1 | 3260.5 |
| SLFN-VA64 (1:1) | 55 | 47.5 | 58.4 | 48.9 7 | 31.97 | 22.5 | 15.56 | 13.45 | 10.6 | 4847.4 |
| SLFN-VA64 (1:1.5) | 85 | 34.2 | 40.1 | 33.5 | 24.8 | 21.7 | 13.8 | 12.6 | 10.2 | 4042.0 |
| SLFN-VA64 (1:2) | 130 | 23.6 | 35.9 | 32.4 | 23.7 | 22.3 | 14.1 | 12.7 | 10.1 | 3924.3 |

| | | | | | | | | | | |
|---|---|---|---|---|---|---|---|---|---|---|
| Note: The disintegration time here was the time for the tablet to disintegrate/dissolve completely in the dissolution cup. The same below. | | | | | | | | | | |

It can be seen based on the above results that the disintegration time of the tablet was significantly prolonged as the amount of VA64 increased. Due to the prolonged disintegration time caused by high VA64 amount, the dissolution did not increase linearly with the increase of VA64 ratio, but first increased and then decreased, and the optimal amount was 1:1. Although the cumulative dissolution of different solid dispersion tablets within 15 min of dissolution was significantly higher than that of the original Nexavar, it decreased rapidly in 15 min ~ 60 min. That is to say, sorafenib drug crystallized and precipitated out immediately after dissolution, and the cumulative dissolution at 60 min~120 min was not significantly different from that of Nexavar tablet.

### 2. Screening of composite carriers

Solid dispersions were prepared according to the ratio in Table 9.

**Table 9 DSC test results of solid dispersions prepared with different composite carriers and main drugs**

| SD formula No. | Main drug | Carrier | | Ratio | Endothermic peak of crystalline drugs in DSC curve |
|---|---|---|---|---|---|
| 1 | SLFN | VA64 | HPMCAS 126G | 1:0.5:1 | Disappeared |
| 2 | SLFN | VA64 | HPMCAS 126G | 1:0.5:0.5 | Disappeared |
| 3 | SLFN | VA64 | HPMCAS 126G | 1:1:0.5 | Disappeared |
| 4 | SLFN | VA64 | HPMCAS 126G | 1:1:0.25 | Disappeared |
| 5 | SLFN | VA64 | HPMCAS 126G | 1:1:0.125 | Disappeared |
| 6 | SLFN | VA64 | HPMCAS 126G | 1:2:0.2 | Disappeared |
| 7 | SLFN | VA64 | HPMCAS 716G | 1:1:0.25 | Disappeared |
| 8 | SLFN | VA64 | HPMC E5 | 1:1:0.25 | Disappeared |
| 9 | SLFN | VA64 | TPGS1000 | 1:1:0.25 | Disappeared |
| 10 | SLFN | VA64 | Tween 80 | 1:1:0.1 | Disappeared |
| 11 | SLFN | HPMC E5 | HPMCAS 126G | 1:1:0.25 | Disappeared |

The solid dispersions prepared with the above different carrier ratios were tableted according to the formula shown in Table 10.

**Table 10 Sorafenib solid dispersible tablet formula**

| Composition of the formula | Amount (mg/tablet) |
|---|---|
| SD amount (based on SLFN) | 100 |
| Silicified microcrystalline cellulose (SMCC) | 218.50 |
| Sodium dodecyl sulfate (SDS) | 4.50 |
| Croscarmellose sodium (CCNa) | 22.50 |
| Magnesium stearate (MS) | 4.50 |

Raw and adjuvant materials in the amount shown in the formula were weighed. All raw and adjuvant materials except for MS were passed through a 50-mesh sieve 6 times, mixed, and granulated by a dry method. The weight of the granules was weighed, and the amount of MS added externally was calculated and weighed. They were mixed for 5 min and subjected to tableting. The dissolution profile was determined and shown in FIG. 2.

Dissolution method: paddle method, pH 6.8 phosphate buffer solution + 0.1% SDS, 900 mL, 100 rpm, 37°C.

Sampling and sample treatment method: 10 mL of sample was taken and filtered with a PES filter membrane (diameter 25 mm, pore size 0.45 µm). 7 mL of filtrate was discarded, 2~3 mL of subsequent filtrate was taken, and 10 mL of medium was added to the dissolution cup. 1 ml of the filtered sample was transferred with a pipette into a 10 ml volumetric flask, and diluted by 10 times by adding a mobile phase to the scale mark.

**Table 11 Dissolution profile of tablets prepared with a composite carrier SD (n=3)**

| SD formula No. | Disintegratio n time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| 1 | 28 | 33.7 | 31.2 | 25.4 | 22.1 | 20.9 | 20.6 | 18.7 | 18.4 | 4906.5 |
| 2 | 26 | 35.9 | 31.5 | 25.2 | 21.3 | 16.1 | 15.3 | 13.7 | 12.8 | 3930.0 |
| 3 | 43 | 42.1 | 38.1 | 29.6 | 26.4 | 20.9 | 17.8 | 16.7 | 14.2 | 4677.0 |
| 4 | 35 | 57.8 | 43.4 | 22.6 | 18.8 | 16.5 | 15.6 | 14.7 | 13.1 | 4168.8 |
| 5 | 28 | 31.2 | 23.6 | 17.9 | 16.9 | 15.4 | 13.2 | 12.3 | 11.5 | 3363.3 |
| 6 | 145 | 28.3 | 25.4 | 14.7 | 18.4 | 17.1 | 16.3 | 14.8 | 14.3 | 3787.8 |
| 7 | 33 | 55.9 | 45.2 | 23.7 | 17.9 | 16.2 | 14.3 | 14.1 | 13.3 | 4096.0 |
| 8 | 54 | 33.7 | 25.4 | 18.9 | 11.2 | 10.1 | 9.6 | 9.1 | 8.7 | 2692.8 |
| 9 | 49 | 34.2 | 37.3 | 20.1 | 13.5 | 11.7 | 10.4 | 9.8 | 9.3 | 2966.0 |
| 10 | 41 | 39.3 | 36.1 | 20.1 | 13.4 | 10.6 | 9.6 | 9.1 | 9 | 2877.5 |
| 11 | 58 | 34.8 | 24.9 | 14.8 | 13.2 | 12.5 | 10.7 | 9.4 | 8.2 | 2715.8 |

The above tablets were sealed in an aluminum foil bag and placed in 60°C for investigation for 10 days, and related substances were detected by HPLC method. The results are shown in Table 12 below.

**Table 12 Test results of related substances in tablets prepared from solid dispersions of different composite carriers at a high temperature of 60°C**

| SD formula No. | Investigation time | Related substances (%) | | |
|---|---|---|---|---|
| | | Maximum single impurity | CTF -aniline | Total impurity |
| 1 | 0 day | 0.04 | Not detected | 0.10 |
| | 10 days | 0.11 | 0.10 | 0.34 |
| 2 | 0 day | 0.04 | Not detected | 0.13 |
| | 10 days | 0.11 | 0.06 | 0.31 |
| 3 | 0 day | 0.04 | Not detected | 0.10 |
| | 10 days | 0.04 | 0.03 | 0.27 |
| 4 | 0 day | 0.04 | Not detected | 0.12 |
| | 10 days | 0.05 | 0.01 | 0.20 |
| 5 | 0 day | 0.04 | Not detected | 0.10 |
| | 10 days | 0.05 | 0.01 | 0.20 |
| 6 | 0 day | 0.04 | Not detected | 0.10 |
| | 10 days | 0.04 | 0.01 | 0.22 |
| 7 | 0 day | 0.04 | Not detected | 0.11 |
| | 10 days | 0.05 | 0.02 | 0.28 |
| 8 | 0 day | 0.04 | Not detected | 0.10 |
| | 10 days | 0.05 | Not detected | 0.18 |
| 9 | 0 day | 0.04 | Not detected | 0.10 |
| | 10 days | 0.05 | 0.01 | 0.21 |
| 10 | 0 day | 0.04 | Not detected | 0.12 |
| | 10 days | 0.05 | 0.02 | 0.24 |
| 11 | 0 day | 0.05 | Not detected | 0.12 |
| | 10 days | 0.08 | 0.05 | 0.34 |

It can be seen from the above data that as for the carrier VA64+HPMCAS, with the increase of the amount of HPMCAS, the dissolution decreased slowly in the later stage, but the stability became poor. Considering the dissolution results and chemical stability results comprehensively, the optimal ratio was 1:1:0.25. The effect was not obvious for other composite carriers.

### Example 4 Screening of crystallization inhibitor

An appropriate amount of sorafenib or sorafenib tosylate was weighed, respectively, dissolved by adding dimethyl sulfoxide and diluted to prepare a high-concentration stock solution containing about 15 mg of sorafenib per 1 ml.

Taking a pH 6.8 phosphate buffer solution containing 0.1% sodium dodecyl sulfate (SDS) as a base medium (pH 6.8 phosphate + 0.1% SDS), an appropriate amount of a polymer carrier polyvinylpyrrolidone (PVP) such as PVP K25, PVP K30, PVP K90, a vinylpyrrolidonevinyl acetate copolymer, such as PVP VA64, hydroxypropyl methylcellulose acetate succinate (HPMCAS), such as HPMCAS HG (or 126 G), HPMCAS MG (912 G), HPMCAS LG (or 716 G), hydroxypropyl methylcellulose, such as HPMC K100LV, polyethylene glycol, such as PEG 1000, PEG 3350, PEG 4000, PEG 6000, and PEG 8000, sodium glycocholate (SGC), sodium taurocholate (STC), sodium deoxycholate (SDC), sodium glycodeoxycholate (SGDC), sodium glycochenodeoxycholate (SGCDC), sodium glycoursodeoxycholate (SGUDC), sodium taurodeoxycholate (STDC) and sodium tauroursodeoxycholate (STUDC), as well as sodium dodecyl sulfonate (SDS), etc. were weighed respectively, and dissolved respectively with pH 6.8 phosphate + 0.1% SDS to prepare a polymer carrier medium containing about 0.5% of the above polymer carrier or a medium containing 0.3% cholate per 1 ml, for later use.

50 ml of the above polymer carrier medium was measured out respectively, placed in a 100 ml stoppered test tube, and shaken in a shaker at 37°C for 1 hour. 0.4 ml of sorafenib stock solution was then added to each stoppered test tube, which was transferred to a constant temperature shaking shaker at 37°C after ultrasonically dispersed uniformly at 37°C. Samples were taken at 0.5h, 1h, 2h, 3h, 4h and 6h, and the concentration of sorafenib in each polymer carrier medium was detected by HPLC method. The results are shown in the Table below.

**Table 13 Screening of polymer carriers, cholates and surfactant type crystallization inhibitors**

| Sampling time | Sorafenib content detection (ug/ml) | | | | | |
|---|---|---|---|---|---|---|
| | 0.5h | 1h | 2h | 3h | 4h | 6h |
| Base medium | 76.48 | 76.99 | 61.16 | 19.39 | 8.34 | 5.30 |
| 0.5% HPMCAS H (126G) | 75.84 | 75.66 | 75.96 | 76.08 | 76.41 | 76.46 |
| 0.5% HPMCAS M | 76.43 | 76.18 | 76.65 | 76.11 | 76.83 | 76.88 |
| 0.5% HPMCAS L (716G) | 76.36 | 76.20 | 77.25 | 79.22 | 76.97 | 76.98 |
| 0.5% PVP K25 | 77.53 | 79.71 | 78.19 | 78.25 | 79.41 | 78.66 |
| 0.5% PVP K30 | 75.71 | 76.02 | 76.22 | 76.20 | 76.78 | 77.33 |
| 0.5% PVP K90 | 74.40 | 75.53 | 75.12 | 74.76 | 75.79 | 77.04 |
| 0.5% PVP-VA64 | 76.69 | 76.34 | 76.65 | 76.79 | 76.97 | 77.51 |
| 0.5% PEG 1000 | 75.66 | 70.78 | 20.46 | 10.69 | 9.06 | 8.43 |
| 0.5% PEG 3350 | 23.97 | 15.34 | 12.52 | 11.74 | 11.57 | 11.70 |
| 0.5% PEG 4000 | 49.17 | 26.27 | 14.35 | 12.76 | 11.87 | 11.69 |
| 0.5% PEG 8000 | 74.01 | 70.07 | 58.32 | 39.73 | 28.13 | 19.01 |
| 0.5% HPMC K100 LV | 14.22 | 11.86 | 11.43 | 10.77 | 10.25 | 8.27 |
| 0.3%SGDC | 15.78 | 6.28 | 4.11 | 4.01 | 4.04 | 3.87 |
| 0.3%SGCDC | 61.59 | 47.08 | 10.41 | 5.38 | 4.28 | 3.68 |
| 0.3%STC | 77.62 | 74.69 | 44.23 | 21.29 | 13.70 | 8.69 |
| 0.3%STDC | 47.72 | 25.90 | 11.19 | 7.82 | 6.58 | 5.90 |
| 0.3%STCDC | 14.43 | 6.22 | 4.69 | 4.79 | 4.81 | 5.79 |
| 0.4%SDS | 76.31 | 69.80 | 47.08 | 34.46 | 29.92 | 24.24 |
| 0.3%SDC | 14.98 | 8.88 | 5.37 | 4.56 | 4.04 | 4.94 |
| 0.2%VA-64+0.3%SDC | 77.73 | 77.86 | 77.77 | 78.04 | 77.96 | 77.84 |
| 0.2%VA-64+0.4%SDS | 77.87 | 77.84 | 77.43 | 77.40 | 77.31 | 77.10 |
| 0.3%SGC | 14.32 | 5.88 | 4.32 | 3.98 | 3.77 | 3.57 |
| 0.2%VA-64+0.3%SGC | 77.82 | 77.85 | 77.84 | 77.92 | 77.96 | 77.83 |
| 0.2%VA-64+0.3%SGCDC | 78.01 | 77.98 | 77.95 | 77.89 | 77.88 | 77.86 |
| 0.2%VA-64+0.3%STDC | 77.56 | 77.67 | 77.88 | 77.89 | 77.83 | 77.38 |

According to the above screening results, polyvinylpyrrolidone series and hydroxypropyl methylcellulose acetate succinate (HPMCAS) series polymer carriers had a significant crystallization inhibition effect, and the crystallization inhibition effect did not decrease with the extension of time. PEG 1000 and PEG 8000 had a good crystallization inhibition effect within 1 hour, but the crystallization became serious with the extension of time. HPMC K100 LV, PEG 3350 and PEG4000 basically had no crystallization inhibition effect.

Cholate is a physiological surfactant secreted by human bile into the intestinal tract. When the preparation made of the pharmaceutical composition containing PVP-VA64 is taken orally, it enters the intestinal tract, and is mixed with cholate with the peristalsis of the gastrointestinal tract, which will not lead to rapid crystallization and precipitation of drugs.

### Example 5 Screening of formula of crystallization inhibitors

### 1. Screening of a crystallization inhibitor in SLFN-VA64 (1:1) SD tablet formula

(1) A polymer carrier crystallization inhibitor/cosolvent was screened according to Table 13 below, and was compared with the PVP series crystallization inhibitor.

**Table 14 Screening formula of crystallization inhibitors**

| Composition of formula | | Amount (mg/tablet) |
|---|---|---|
| Material added internally | SLFN-SD (based on the total amount of SLFN and VA64) | 200.00 |
| | SMCC | 126.00 |
| | Crystallization inhibitor /cosolvent | 75.00 |
| | CCNa | 45.00 |
| | MS | 3.00 |
| Material added externally | SMCC | 88.00 |
| | SiO₂ | 60.00 |
| | MS | 3.00 |

Among them, the mass ratio of SLFN to VA64 was 1:1.

Crystallization inhibitor/cosolvent: HPMC E5, HPMC K4M, polyoxyethylene hydrogenated castor oil RH40, xanthan gum, TPGS 1000, SOLUPLUS, PVP K25, PVP K30, and HPMCAS 126G.

Preparation process: raw and adjuvant materials in the amount shown in the formula were weighed. Raw and adjuvant materials added internally were passed together through a 50-mesh sieve 6 times, mixed, and granulated by a dry method. The weight of the granules was weighed, and the amount of material added externally was calculated and weighed. They were mixed for 5 min and subjected to tableting, and cumulative dissolution was tested.

The dissolution method was the same as before.

**Table 15 Cumulative dissolution of different crystallization inhibitor/cosolvent formulas (n=3)**

| SD No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| PVP K25 | 2 | 90.3 | 70.5 | 49.6 | 40.2 | 35.4 | 31.3 | 28.1 | 27.6 | 8002.5 |
| PVP K30 | 2 | 88.9 | 68.9 | 46.3 | 37.3 | 32.7 | 28.1 | 25.4 | 24.3 | 7554.3 |
| HPMC E5 | 8 | 49.2 | 50.1 | 33.6 | 25.8 | 21.3 | 17.9 | 14.2 | 12.9 | 4617.8 |
| HPMC K4M | 39 | 33.7 | 40.1 | 41.5 | 30.1 | 24.6 | 20.4 | 18.2 | 14.6 | 5245.0 |
| RH40 | 68 | 29.5 | 33.4 | 35.4 | 28.6 | 27.1 | 20.5 | 14.6 | 11.9 | 4647.5 |
| Xanthan gum | 24 | 39.4 | 40.8 | 30.4 | 28.7 | 25.3 | 20.6 | 18.5 | 16.9 | 5202.8 |
| TPGS1000 | 55 | 29.3 | 44.2 | 40.1 | 33.4 | 20.6 | 18.7 | 14.3 | 13.2 | 4733.0 |
| SOLUPLUS | 13 | 38.9 | 49.8 | 50.1 | 30.3 | 18.9 | 16.4 | 14.2 | 12.8 | 4872.8 |
| HPMCAS126G | 15 | 79.3 | 54.6 | 31.1 | 24.5 | 21.5 | 18.6 | 17.4 | 15.7 | 5166.0 |

It can be seen from the above results that the crystallization inhibitors PVPK 30 and PVP K25 were obviously superior to other cosolvents/ crystallization inhibitors.

The above tablets were sealed in aluminum foil bags, and were investigated for influencing factors at a high temperature of 60°C. Related substances were detected by HPLC, and the physical stability was detected by DSC. The results are shown in Table 15 below:

**Table 16 Stability results of solid dispersion tablets added with different crystallization inhibitors at a high temperature of 60°C**

| Crystallization inhibitor | Time | Related substances (%) | | | Endothermic peak of crystalline drugs in DSC curve |
|---|---|---|---|---|---|
| | | Maximum single impurity | CTF -aniline | Total impurity | |
| PVP K30 | 0 day | 0.04 | Not detected | 0.10 | No |
| | 10 days | 0.04 | Not detected | 0.13 | No |
| PVP K25 | 0 day | 0.04 | Not detected | 0.10 | No |
| | 10 days | 0.04 | Not detected | 0.12 | No |
| HPMC E5 | 0 day | 0.04 | Not detected | 0.10 | No |
| | 10 days | 0.05 | 0.01 | 0.18 | No |
| HPMC K4M | 0 day | 0.04 | Not detected | 0.10 | No |
| | 10 days | 0.04 | 0.01 | 0.17 | No |
| RH40 | 0 day | 0.04 | Not detected | 0.11 | No |
| | 10 days | 0.05 | 0.03 | 0.26 | Yes |
| Xanthan gum | 0 day | 0.04 | Not detected | 0.10 | No |
| | 10 days | 0.04 | 0.01 | 0.26 | No |
| TPGS1000 | 0 day | 0.04 | Not detected | 0.09 | No |
| | 10 days | 0.05 | Not detected | 0.19 | Yes |
| SOLUPLUS | 0 day | 0.04 | Not detected | 0.10 | No |
| | 10 days | 0.04 | Not detected | 0.14 | No |
| HPMCAS 126G | 0 day | 0.04 | Not detected | 0.09 | No |
| | 10 days | 0.04 | 0.03 | 0.25 | No |

From the above data, it can be seen that the low-melting cosolvent underwent crystal transformation of the main drug during placement, resulting in a poor physical stability.

### (2) Screening of cholate crystallization inhibitors

**Table 17 Screening formulas of crystallization inhibitors**

| Composition of formula | | Amount (mg/tablet) |
|---|---|---|
| Material added internally | SLFN-SD (based on the total amount of SLFN and VA64) | 200.00 |
| | SMCC | 126.00 |
| | CCNa | 45.00 |
| | MS | 3.00 |
| Material added externally | SMCC | 88.00 |
| | Crystallization inhibitor | 75.00 |
| | SiO₂ | 60.00 |
| | MS | 3.00 |

Among them, the mass ratio of SLFN to VA64 was 1:1.

Crystallization inhibitor/cosolvent: SGC, SDC.

Preparation process: raw and adjuvant materials in the amount shown in the formula were weighed. Raw and adjuvant materials added internally were passed together through a 50-mesh sieve 6 times, mixed, and granulated by a dry method. The weight of the granules was weighed, and the amount of material added externally was calculated and weighed. They were mixed for 5 min and subjected to tableting, and cumulative dissolution was tested.

The dissolution method was the same as before. Comparison was made with the formula of PVPK25 as the crystallization inhibitor.

**Table 18 Dissolution results of SLFN-VA64 solid dispersion-cholate crystallization inhibitor (n=3)**

| SD No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| PVP K25 | 2 | 90.3 | 70.5 | 49.6 | 40.2 | 35.4 | 31.3 | 28.1 | 27.6 | 8002.5 |
| SGC | 4 | 51.6 | 69.6 | 80.3 | 60.8 | 35.1 | 17.1 | 13.3 | 12.1 | 6399.9 |
| SDC | 5 | 45.0 | 66.2 | 82.9 | 85.0 | 65.0 | 17.7 | 9.6 | 9.1 | 6942.5 |

According to the above results, in the case of the formula with VA-64 as a carrier and the external addition of cholates, the dissolution of sorafenib solid dispersion tablets within 60 min was greatly improved, but decreased to the same level as Nexavar after 60 min, and the time to maintain the crystal inhibition effect was not as long as that of the PVP series.

2. Screening of crystallization inhibitors in tablets prepared from solid dispersions with SLFN-VA64-HPMCAS (1:1:0.25) as a carrier

The crystallization inhibitors/cosolvents were screened according to Table 19 below.

**Table 19 Screening of crystallization inhibitors - carrier SLFN-VA64-HPMCAS (1:1:0.25)**

| Composition of formula | | Amount (mg/tablet) |
|---|---|---|
| Material added internally | SLFN-SD (based on the total amount of SLFN-VA64-HPMCAS) | 225 |
| | MCC101 | 69.3 |
| | CaSO4 | 34.7 |
| | Crystallization inhibitor | 80 |
| | CCNa | 45 |
| | MS | 3 |
| Material added externally | MCC102 | 80 |
| | SiO2 | 60 |
| | MS | 3 |

Preparation process: raw and adjuvant materials in the amount shown in the formula were weighed. Raw and adjuvant materials added internally were passed together through a 50-mesh sieve 6 times, mixed, and granulated by a dry method. The weight of the granules was weighed, and the amount of material added externally was calculated and weighed. They were mixed for 5 min and subjected to tableting, and the dissolution profile was determined.

The dissolution method was the same as before.

**Table 20 Dissolution profiles of different crystallization inhibitor formulas (n=3)**

| Different crystallization inhibitors | Disintegration time min | Cumulative dissolution (%) | | | | | | | | AUC0-4h |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| PVP K30 | 3 | 75.7 | 89.5 | 71.2 | 55.4 | 47.6 | 41.0 | 37.8 | 33.3 | 10582.3 |
| PVP K25 | 2 | 78.2 | 92.6 | 75.4 | 57.6 | 50.1 | 43.8 | 39.5 | 35.1 | 11248.1 |
| HPMC K4M | 28 | 22.9 | 56.4 | 43.9 | 30.8 | 23.4 | 19.3 | 17.1 | 15.7 | 5233.5 |
| Xanthan gum | 21 | 39.4 | 43.8 | 36.7 | 29.5 | 21.5 | 18.1 | 17.2 | 16.2 | 5071.5 |
| SOLUPLUS | 11 | 59.4 | 42.6 | 40.2 | 27.1 | 21.3 | 19.2 | 16.8 | 15.4 | 5208.8 |

From the above results, it can be seen that the crystallization inhibition effects of PVP K25 and PVPK30 were superior to that of the other three crystallization inhibitors.

### Example 6 screening of preparation formula

### 5.1. Effects of silica on disintegration and dissolution of preparations

Tablets were prepared according to the formula shown in Table 21. The preparation method was the same as before.

**Table 21 Investigation formula of the amount of silica**

| Composition of formula | | Amount of raw and adjuvant materials/tablet (mg) | | | | |
|---|---|---|---|---|---|---|
| | | Formula 1 | Formula 2 | Formula 3 | Formula 4 | Formula 5 |
| Material added internally | SLFN-SD (Based on the total amount of SLFN and VA64) | 200.00 | 200.00 | 200.00 | 200.00 | 200.00 |
| | SMCC | 126.00 | 126.00 | 126.00 | 126.00 | 126.00 |
| | PVP K30 | 75.00 | 75.00 | 75.00 | 75.00 | 75.00 |
| | CCNa | 45.00 | 45.00 | 45.00 | 45.00 | 45.00 |
| | MS | 3.00 | 3.00 | 3.00 | 3.00 | 3.00 |
| Material added externally | SMCC | 88.00 | 88.00 | 88.00 | 88.00 | 88.00 |
| | SiO₂ | / | 6 | 12 | 30 | 90 |
| | MS | 3.00 | 3.00 | 3.00 | 3.00 | 3.00 |

Among them, the mass ratio of SLFN to VA64 was 1:1.

The dissolution results are shown in Table 22.

**Table 22 Effects of different SiO₂ on dissolution (n=3)**

| SD No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| Formula 1 | 35 | 33.4 | 40.7 | 35.8 | 25.2 | 23.1 | 21.2 | 18.3 | 17.4 | 5209.8 |
| Formula 2 | 28 | 38.2 | 43.6 | 36.7 | 26.4 | 22.8 | 20.7 | 18.9 | 17.3 | 5315.8 |
| Formula 3 | 16 | 51.1 | 52.4 | 37.8 | 29.5 | 26.1 | 23.4 | 21.5 | 19.8 | 6028.0 |
| Formula 4 | 13 | 67.4 | 54.2 | 40.1 | 31.5 | 27.6 | 23.7 | 22.6 | 20.2 | 6390.3 |
| Formula 5 | 2 | 88.4 | 71.2 | 50.6 | 36.8 | 31.4 | 27.8 | 24.3 | 23.7 | 7494.5 |

From the above results, it can be seen that with the increase of the amount of silica, the disintegration became faster and the dissolution improved.

### 5.2. Investigation of fillers

Tablets were prepared according to the formula shown in Table 23. The preparation method was the same as before.

**Table 23 Investigation formula of the amount of fillers**

| Composition of formula | | Amount of raw and adjuvant materials in each formula/tablet (mg) | | | | |
|---|---|---|---|---|---|---|
| | | Formula 6 | Formula 7 | Formula 8 | Formula 9 | Formula 10 |
| Material added internally | SLFN-SD (based on the total amount of SLFN-VA64-HPMCAS) | 225 | 225 | 225 | 225 | 225 |
| | MCC101 | 69.3 | 69.3 | 69.3 | 69.3 | 79.3 |
| | Mannitol | 34.7 | / | / | / | / |
| | Pregelatinized starch | / | 34.7 | / | / | / |
| | Lactose | / | / | 34.7 | / | / |
| | Calcium hydrogen phosphate | / | / | / | 34.7 | / |
| | Starch | / | / | / | / | 24.7 |
| | PVPK30 | 80 | 80 | 80 | 80 | 80 |
| | CCNa | 45 | 45 | 45 | 45 | 45 |
| | MS | 3 | 3 | 3 | 3 | 3 |
| Material added externally | MCC102 | 80 | 80 | 80 | 80 | 80 |
| | SiO₂ | 60 | 60 | 60 | 60 | 60 |
| | MS | 3 | 3 | 3 | 3 | 3 |

Among them, the mass ratio of SLFN-VA64-HPMCAS was 1:1:0.25.

The dissolution results are shown in Table 24 below:

**Table 24 Effects of different fillers on dissolution (n=3)**

| SD No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| Formula 6 | 6 | 72.1 | 85.4 | 63.2 | 51.9 | 40.3 | 37.2 | 34.5 | 24.6 | 9254.5 |
| Formula 7 | 8 | 70.6 | 81.3 | 55.9 | 47.2 | 41.3 | 32.8 | 29.6 | 25.4 | 8559.0 |
| Formula 8 | 7 | 65.7 | 80.4 | 51.3 | 49.2 | 37.8 | 30.5 | 27.4 | 22.9 | 7981.5 |
| Formula 9 | 5 | 75.7 | 81.9 | 62.3 | 54.3 | 36.5 | 30.4 | 26.8 | 20.7 | 8178.8 |
| Formula 10 | 8 | 77.6 | 80.1 | 51.4 | 46.1 | 34.6 | 28.7 | 25.8 | 23.7 | 7827.0 |

The above results show that different fillers had a slight influence on the dissolution results, but the dissolution was higher compared to the original preparation.

### 5.3 Investigation of disintegrants

Tablets were prepared according to the formula shown in Table 25. The preparation method was the same as before.

**Table 25 Investigation formula of the amount of disintegrants**

| Composition of formula | | Amount of raw and adjuvant materials in each formula/tablet (mg) | | |
|---|---|---|---|---|
| | | Formula 11 | Formula 12 | Formula 13 |
| Material added internally | SLFN-SD (based on the total amount of SLFN-VA64-HPMCAS) | 225 | 225 | 225 |
| | MCC101 | 69.3 | 69.3 | 69.3 |
| | CaSO4 | 34.7 | 34.7 | 34.7 |
| | PVP K30 | 80 | 80 | 80 |
| | PVPP | 45 | / | / |
| | CMS-Na | / | 45 | / |
| | L-HPC | / | / | 90 |
| | MS | 3 | 3 | 3 |
| Material added externally | MCC102 | 80 | 80 | 80 |
| | SiO₂ | 60 | 60 | 60 |
| | MS | 3 | 3 | 3 |

Among them, the mass ratio of SLFN-VA64-HPMCAS was 1:1:0.25.

The dissolution results are shown in Table 26 below:

**Table 26 Effects of different disintegrants on dissolution (n=3)**

| Formula No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| Formula 11 | 2 | 85.4 | 72.3 | 65.7 | 58.7 | 43.6 | 40.5 | 35.7 | 30.6 | 10070.3 |
| Formula 12 | 9 | 80.1 | 71.2 | 55.7 | 43.6 | 38.1 | 30.5 | 25.8 | 22.6 | 7927.3 |
| Formula 13 | 6 | 84.3 | 73.5 | 64.8 | 55.1 | 42 | 30.4 | 23.2 | 21.2 | 8153.3 |

It can be seen from the above results that although there were differences between different disintegrants, the dissolution was all good.

### 5.4 Preparation of samples with different specifications

**Table 27 Formulas of samples with different specifications (100 tablets/batch)**

| Composition of formula | | 70 mg specification | 80 mg specification | 120 mg specification | 140 mg specification | 150 mg specification |
|---|---|---|---|---|---|---|
| | | Amount (mg/tablet) | Amount (mg/tablet) | Amount (mg/tablet) | Amount (mg/tablet) | Amount (mg/tablet) |
| Materi al added interna lly | SD carrier and ratio | SLFN-VA64-HPMCAS (1:1:0.25) | SLFN-VA64-HPMCAS (1:1:0.25) | SLFN-VA64-HPMCAS (1:1:0.25) | SLFN-VA64-HPMCAS (1:1:0.25) | SLFN-VA64-HPMCAS (1:1:0.25) |
| | SLFN-SD | 157.5 | 180.0 | 270.0 | 315.0 | 337.5 |
| | MCC PH101 | 69.3 | 83.2 | 83.2 | 93.2 | 104.0 |
| | CaSO4 | 34.7 | 41.6 | 41.6 | 51.6 | 52.1 |
| | PVPK 30 | 80 | / | 96 | / | 120 |
| | PVPK 25 | / | 90 | / | 110 | / |
| | CCNa | 45 | 54 | 54 | 64 | 67.5 |
| | MS | 3 | 3.6 | 3.6 | 4.2 | 4.5 |
| Materi al added externa lly | MCC PH102 | 80 | 96 | 96 | 110 | 120 |
| | SiO₂ | 60 | 72 | 72 | 85 | 90 |
| | MS | 3 | 3.6 | 3.6 | 4.2 | 4.5 |

Preparation process: raw and adjuvant materials in the amount shown in the formula were weighed. Raw and adjuvant materials added internally were passed together through a 50-mesh sieve 6 times, mixed, and granulated by a dry method. The weight of the granules was weighed, and the amount of material added externally was calculated and weighed. They were mixed for 5 min and subjected to tableting.

The dissolution results are shown in Table 28 below:

**Table 28 Dissolution results of samples with different specifications (n=3)**

| Formula No. | Disintegration time min | Cumulative dissolution (%) | | | | | | | | AUC₀₋₄ₕ |
|---|---|---|---|---|---|---|---|---|---|---|
| | | 5min | 15 min | 30 min | 45 min | 60 min | 90 min | 120 min | 240 min | |
| Nexavar (half tablet, 100 mg) | 4 | 14.6 | 16.4 | 14.6 | 13.1 | 12.4 | 11.1 | 10.2 | 9.9 | 2687.5 |
| 70 mg | 3 | 99.8 | 99.9 | 98.2 | 90.4 | 75.8 | 66.4 | 59.9 | 52.1 | 16129.5 |
| 80 mg | 3 | 99.7 | 99.6 | 96.1 | 90.8 | 77.3 | 71.5 | 61.4 | 55 | 16559.3 |
| 120 mg | 11 | 80.3 | 71.6 | 63.7 | 55.8 | 40.8 | 35.9 | 33.6 | 30.3 | 9563.5 |
| 140 mg | 11 | 73.1 | 65.2 | 60.8 | 50.4 | 37.7 | 33.4 | 32.8 | 27.6 | 8964.5 |
| 150 mg | 13 | 66.4 | 55.1 | 50.8 | 44.9 | 35.7 | 31.6 | 29.7 | 25.4 | 8092.8 |

It can be seen that as the specification increased, the dissolution percentage decreased slightly, but it was much higher than that of the reference preparation Nexavar.

### Example 7 Comparison of animal experiments

### 6.1 Preparation of animal experimental samples

**Table 29 Formulas of animal experimental samples (1000 tablets/batch)**

| Composition of formula | | 20200509-1 | 20200509-2 | 20200509-3 |
|---|---|---|---|---|
| | | Amount (mg/tablet) | Amount (mg/tablet) | Amount (mg/tablet) |
| Material added internally | SD carrier and ratio | SLFN-VA64 (1:1) | SLFN-VA64 (1:1) | SLFN-VA64-HPMCAS (1:1:0.25) |
| | SLFN-SD | 200.00 | 200.00 | 225 |
| | SMCC | 126.00 | 126.00 | / |
| | MCC PH101 | / | / | 69.3 |
| | CaSO4 | / | / | 34.7 |
| | PVPK30 | 75.00 | / | 80 |
| | HPMCE5 | / | 75.00 | / |
| | CCNa | 45.00 | 45.00 | 45 |
| | MS | 3.00 | 3.00 | 3 |
| Material added externally | SMCC | 88.00 | 88.00 | / |
| | MCC PH102 | / | / | 80 |
| | SiO₂ | 60.00 | 60.00 | 60 |
| | MS | 3.00 | 3.00 | 3 |

Preparation process: raw and adjuvant materials in the amount shown in the formula were weighed. Raw and adjuvant materials added internally were passed together through a 50-mesh sieve 6 times, mixed, and granulated by a dry method. The weight of the granules was weighed, and the amount of material added externally was calculated and weighed. They were mixed for 5 min and subjected to tableting.

The above samples (specification 100 mg) and a reference preparation Nexavar (specification 200 mg) were used for animal experiments.

Animals: adult male Beagle (8-11 kg), which can be non-naïve; they were divided into 4 groups with 2 in each group.

Administration route and administration frequency: single oral administration of 1 tablet by gavage, fasting for 16 hours or more before administration, and free feeding after 4 hours of administration; the cleaning period was 4 days. 4 cycles of cross administration.

Blood sampling time points: 0, 1.5, 2, 2.5, 3, 4, 5, 6, 8, 12, 24, and 48 h.

LC/MS/MS method was used to detect blood concentration, and Phoenix WinNonlin 7.0 was used to calculate pharmacokinetic parameters (AUC₀₋ₜ, AUC_{0-∞}, Cₘₐₓ, T_{1/2}, Tₘₐₓ and arithmetic mean (±SD) and geometric mean of these parameters).

**Table 30 Experimental results of Beagles**

| No. | Cmax (ng/ml) | | | | AUC0→t(ng/ml*h) | | | |
|---|---|---|---|---|---|---|---|---|
| | Reference | 20200509-1 | 20200509-2 | 20200509-3 | Reference | 20200509-1 | 20200509-2 | 20200509-3 |
| dog-#1 | 2103.3 | 3151.4 | 820.5 | 2987.3 | 11744.5 | 18830.6 | 4774.1 | 16656.2 |
| dog-#2 | 3727.9 | 1300.5 | 1289.7 | 2138.7 | 21184.8 | 9465.3 | 9038.7 | 12778.1 |
| dog-#3 | 1147.9 | 1410.4 | 1402.5 | 3210.1 | 9054.1 | 8596.5 | 8623.8 | 18922.1 |
| dog-#4 | 3038.3 | 3287.2 | 2587.5 | 2463.3 | 17725.1 | 19089.2 | 13543.5 | 12931.2 |
| dog-#5 | 2352.6 | 2660.1 | 2634.5 | 2180.5 | 12350.7 | 14397.4 | 12508.6 | 11207.4 |
| dog-#6 | 934.8 | 2304.7 | 1388.1 | 2003.3 | 7418.3 | 13382.7 | 9665.9 | 10532.2 |
| dog-#7 | 1904.3 | 1986.3 | 676.8 | 2507.5 | 10367.9 | 10888.7 | 3728.7 | 17340.5 |
| dog-#8 | 856.7 | 1355.0 | 1050.8 | 1049.3 | 6124.3 | 9679.5 | 6705.1 | 9331.9 |
| Mean | 2008.2 | 2182.0 | 1481.3 | 2317.5 | 11996.2 | 13041.2 | 8573.6 | 13712.5 |
| CV | 51.1 | 36.7 | 50.2 | 12.5 | 42.8 | 31.8 | 40.2 | 25.6 |
| Geometric mean | 1774.052 | 2051.327 | 1333.31 | 2215.58 | 11111.03 | 12494.55 | 7904.149 | 13326.41 |
| R test/ reference | / | 1.16 | 0.75 | 1.25 | / | 1.12 | 0.71 | 1.20 |

It can be seen from the above results that 20200509-1 and 20200519-3 were significantly higher than 20200509-2. The 100 mg dosage of the two was basically equivalent to 200 mg of the reference preparation. Comparing the CVs of the reference and the test sample, it can be seen that 20200509-1 and 20200509-3 were significantly better than the reference preparation.

The description of the above examples is only used to help understand the method of the present disclosure and core idea thereof. It should be noted that for those of ordinary skill in the art, several improvements and modifications can be made to the present disclosure without departing from the principles of the present disclosure, and these improvements and modifications also fall within the protection scope of the claims of the present disclosure.

## Claims

1. A sorafenib pharmaceutical composition with high bioavailability, comprising:
a) sorafenib, or a salt, hydrate, solvate, or a hydrate or solvate of the salt thereof; and
b) a carrier comprising VA64 and HPMCAS.

2. The sorafenib pharmaceutical composition according to claim 1, wherein the mass ratio of the component a) to VA64 and HPMCAS in component b) is 1:(0.1~5):(0.01~5), preferably 1:(0.5~3):(0.1~1).

3. The sorafenib pharmaceutical composition according to claim 1, wherein the mass ratio of the component a) to VA64 and HPMCAS in component b) is 1:1:(0.1∼0.5), preferably 1:1:0.25.

4. The sorafenib pharmaceutical composition according to claim 1, wherein the sorafenib pharmaceutical composition is a solid dispersion, the solid dispersion is prepared according to a spray drying method, and the solvent of the spray drying method is a mixed solvent of methanol and dichloromethane.

5. The sorafenib pharmaceutical composition according to claim 4, wherein the volume ratio of methanol and dichloromethane is 1:(1~4), preferably 1:3.

6. A low-dose sorafenib oral solid preparation, comprising:
a) a sorafenib solid dispersion;
b) a crystallization inhibitor; and
c) additional pharmaceutically acceptable adjuvant.

7. The low-dose sorafenib oral solid preparation according to claim 6, wherein the sorafenib solid dispersion comprises:
a) sorafenib, or a salt, hydrate, solvate, or a hydrate or solvate of the salt thereof; and
b) a carrier comprising VA64.

8. The low-dose sorafenib oral solid preparation according to claim 6, wherein the sorafenib solid dispersion comprises:
a) sorafenib, or a salt, hydrate, solvate, or a hydrate or solvate of the salt thereof; and
b) a carrier comprising VA64 and HPMCAS.

9. The sorafenib oral solid preparation according to claim 6, wherein the component a) has a unit dose of 70-200 mg, preferably 70~140 mg.

10. The sorafenib oral solid preparation according to claim 6, wherein the crystallization inhibitor is one or more selected from polyvinylpyrrolidone, hydroxypropyl methylcellulose acetate succinate (HPMCAS), sodium glycocholate, sodium taurocholate, sodium glycodeoxycholate, sodium glycochenodeoxycholate, sodium glycoursodeoxycholate, sodium taurodeoxycholate and sodium tauroursodeoxycholate, as well as sodium dodecyl sulfonate.

11. The sorafenib oral solid preparation according to claim 6, wherein the mass content of the crystallization inhibitor is 1%~40%, preferably 1%~20%.

12. The sorafenib oral solid preparation according to claim 6, wherein the adjuvant is one or more selected from a filler, disintegrant, binder, glidant, lubricant, and flavoring agent.

13. The sorafenib oral solid preparation according to claim 12, wherein the filler is one or more selected from mannitol, pregelatinized starch, lactose, calcium hydrogen phosphate, starch, microcrystalline cellulose, pregelatinized starch, partially pregelatinized starch, magnesium sulfate, and calcium sulfate;
the disintegrant is one or more selected from corn starch, partially pregelatinized starch, hydroxypropyl starch, carboxymethyl cellulose, sodium carboxymethyl cellulose, calcium carboxymethyl cellulose, sodium carboxymethyl starch, low-substituted hydroxypropyl cellulose, croscarmellose sodium, and crospovidone;
the binder is one or more selected from hydroxypropyl cellulose, hydroxypropyl methylcellulose, povidone, starch slurry, and sodium carboxymethyl cellulose;
the glidant is one or more selected from talc and silica; and
the lubricant is one or more selected from magnesium stearate, stearic acid, calcium stearate, sodium stearyl fumarate, polyethylene glycol, hydrogenated vegetable oil, polyethylene glycol, sodium dodecyl sulfate, talc, and silica.

14. The sorafenib oral solid preparation according to claim 12, wherein the glidant is silica.

15. The sorafenib oral solid preparation according to claim 13, wherein the mass content of the silica is 2%~20%, preferably 5%~15%, and more preferably 10%.

16. The sorafenib oral solid preparation according to claim 6, wherein the dosage form of the oral solid preparation is tablet, granule, dry suspension, capsule or film.

17. Use of the sorafenib pharmaceutical composition with high bioavailability according to any one of claims 1 to 5 or the sorafenib oral solid preparation according to any one of claims 6 to 16 in the manufacture of a medicament for preventing, treating or alleviating liver cancer, renal cell carcinoma and thyroid cancer.

## Amended claims

### Amended claims under Art. 19.1 PCT

1. A sorafenib pharmaceutical composition with high bioavailability, comprising:
a) sorafenib, or a salt, hydrate, solvate, or a hydrate or solvate of the salt thereof; and
b) a carrier comprising VA64 and HPMCAS;
the mass ratio of the component a) to VA64 and HPMCAS in component b) is 1:(0.5∼3):(0.1∼1).

2. The sorafenib pharmaceutical composition according to claim 1, wherein the mass ratio of the component a) to VA64 and HPMCAS in component b) is 1:1:(0.1∼0.5), preferably 1:1:0.25.

3. The sorafenib pharmaceutical composition according to claim 1, wherein the sorafenib pharmaceutical composition is a solid dispersion, the solid dispersion is prepared according to a spray drying method, and the solvent of the spray drying method is a mixed solvent of methanol and dichloromethane.

4. The sorafenib pharmaceutical composition according to claim 3, wherein the volume ratio of methanol and dichloromethane is 1:(1∼4), preferably 1:3.

5. A low-dose sorafenib oral solid preparation, comprising:
a) a sorafenib solid dispersion;
b) a crystallization inhibitor; and
c) additional pharmaceutically acceptable adjuvant;
the crystallization inhibitor is selected from polyvinylpyrrolidone;
or the crystallization inhibitor is one or more selected from hydroxypropyl methylcellulose acetate succinate (HPMCAS), sodium glycocholate, sodium taurocholate, sodium glycodeoxycholate, sodium glycochenodeoxycholate, sodium glycoursodeoxycholate, sodium taurodeoxycholate and sodium tauroursodeoxycholate, as well as sodium dodecyl sulfonate.

6. The low-dose sorafenib oral solid preparation according to claim 5, wherein the sorafenib solid dispersion comprises:
a) sorafenib, or a salt, hydrate, solvate, or a hydrate or solvate of the salt thereof; and
b) a carrier comprising VA64.

7. The low-dose sorafenib oral solid preparation according to claim 5, wherein the sorafenib solid dispersion comprises:
a) sorafenib, or a salt, hydrate, solvate, or a hydrate or solvate of the salt thereof; and
b) a carrier comprising VA64 and HPMCAS.

8. The sorafenib oral solid preparation according to claim 5, wherein the component a) has a unit dose of 70-200 mg, preferably 70~140 mg.

9. The sorafenib oral solid preparation according to claim 5, wherein the mass content of the crystallization inhibitor is 1%~40%, preferably 1%~20%.

10. The sorafenib oral solid preparation according to claim 5, wherein the adjuvant is one or more selected from a filler, disintegrant, binder, glidant, lubricant, and flavoring agent.

11. The sorafenib oral solid preparation according to claim 10, wherein the filler is one or more selected from mannitol, pregelatinized starch, lactose, calcium hydrogen phosphate, starch, microcrystalline cellulose, pregelatinized starch, partially pregelatinized starch, magnesium sulfate, and calcium sulfate;
the disintegrant is one or more selected from corn starch, partially pregelatinized starch, hydroxypropyl starch, carboxymethyl cellulose, sodium carboxymethyl cellulose, calcium carboxymethyl cellulose, sodium carboxymethyl starch, low-substituted hydroxypropyl cellulose, croscarmellose sodium, and crospovidone;
the binder is one or more selected from hydroxypropyl cellulose, hydroxypropyl methylcellulose, povidone, starch slurry, and sodium carboxymethyl cellulose;
the glidant is one or more selected from talc and silica; and
the lubricant is one or more selected from magnesium stearate, stearic acid, calcium stearate, sodium stearyl fumarate, polyethylene glycol, hydrogenated vegetable oil, polyethylene glycol, sodium dodecyl sulfate, talc, and silica.

12. The sorafenib oral solid preparation according to claim 10, wherein the glidant is silica.

13. The sorafenib oral solid preparation according to claim 12, wherein the mass content of the silica is 2%~20%, preferably 5%~15%, and more preferably 10%.

14. The sorafenib oral solid preparation according to claim 5, wherein the dosage form of the oral solid preparation is tablet, granule, dry suspension, capsule or film.

15. Use of the sorafenib pharmaceutical composition with high bioavailability according to any one of claims 1 to 4 or the sorafenib oral solid preparation according to any one of claims 5 to 14 in the manufacture of a medicament for preventing, treating or alleviating liver cancer, renal cell carcinoma and thyroid cancer.
